**Europäisches Patentamt**

**European Patent Office**

**Office européen des brevets**

(19)

(11) Veröffentlichungsnummer: **0 197 489**
**A2**

(12) # EUROPÄISCHE PATENTANMELDUNG

(21) Anmeldenummer: **86104440.2**

(22) Anmeldetag: **01.04.86**

(51) Int. Cl.⁴: **C 12 N 15/00**
**C 12 N 5/00**

(30) Priorität: **06.04.85 DE 3512559**

(43) Veröffentlichungstag der Anmeldung:
**15.10.86 Patentblatt 86/42**

(84) Benannte Vertragsstaaten:
**AT BE CH DE FR GB IT LI LU NL SE**

(71) Anmelder: **BEHRINGWERKE Aktiengesellschaft**
**Postfach 1140**
**D-3550 Marburg 1(DE)**

(72) Erfinder: **Parwaresch, Reza, Prof. Dr.**
**Düsternbrooker Weg 45**
**D-2300 Kiel(DE)**

(72) Erfinder: **Radzun, Hans Jochen, Dr.**
**Lornsenstrasse 34**
**D-2300 Kiel(DE)**

(72) Erfinder: **Kreipe, Hans, Dr.**
**Wilhelminenstrasse 13**
**D-2300 Kiel(DE)**

(74) Vertreter: **Becker, Heinrich Karl Engelbert, Dr. et al,**
**HOECHST AKTIENGESELLSCHAFT Central Patent**
**Department P.O. Box 80 03 20**
**D-6230 Frankfurt am Main 80(DE)**

(54) **Hypoxanthin-guanin-phosphoribosyltransferase-negative Mutante einer histiozytischen permanenten humanen Zellinie.**

(57) Es wird ein Verfahren zur Herstellung mutierter Zellen der Zellinie U–937 beschrieben, worin Zellen dieser Zellinie mit einem zur Mutierung tierischer Zellen bekannten chemischen Agenz und dann mit 6-Thioguanin-behandelt werden, wobei Zellen erhalten werden, in denen Hypoxyanthin-guanin-phosphoribosyltransferase nicht exprimiert wird.

Diese können als Fusionspartner on Monozyten verwendet werden.

EP 0 197 489 A2

Hypoxanthin-guanin-phosphoribosyltransferase-negative
Mutante einer histiozytischen permanenten humanen Zell-
linie

Die Erfindung betrifft eine permanente humane Zellinie
mit einem Enzymdefekt, die zur Immortalisierung anderer
Zellen verwendet werden kann.

Die Technik der Immortalisierung von Zellen durch Fusion
mit Zellen permanenter Zellinien ist seit langem bekannt. Solche Hybridzellen oder Hybridoma wurden beispielsweise durch die Fusion von Blutzellen mit Tumorzellen erzielt. In dem klassischen Weg, wie er von Köhler und Milstein (Nature 256, 495-497, 1975) beschrieben
wurde, wurden als permanent wachsende Tumorzellen Myelomzellen der Maus mit B-Lymphozyten der Maus fusioniert. Die aus derartigen Fusionen resultierenden
Hybridzellen sind in der Lage, Stoffe zu sekretieren,
die z. B. aus Kulturüberstand derartiger Hybridzellkulturen einfach und in großen Mengen isoliert werden können. Im Falle der Fusion von B-Lymphozyten mit Myelomzellen produzieren die Hybridzellen eine homogene Antikörperpopulation (monoklonale Antikörper), wobei durch
geeignete Selektionsverfahren solche Hybridzellklone
isoliert und weitergezüchtet werden können, die monoklonale Antikörper einer gewünschten Spezifität produzieren.

Welche Stoffe eine Hybridzelle zu produzieren in der
Lage ist, hängt grundsätzlich von den zur Fusion verwendeten Fusionszellpartnern ab. Werden als Fusionspartner
B-Lymphozyten der Maus mit Myelomzellen verschmolzen, so

0197489

sind die von einer derartigen Hybridzelle produzierten Stoffe monoklonale Antikörper murinen Ursprungs, während bei Verwendung von humanen B-Lymphozyten als Fusionspartner von Myelomzellen die daraus resultierenden Hybridzellen Antikörper mit allen Charakteristika von menschlichen Immunglobulinen produzieren, unabhängig davon, ob die verwendete Myelomzelle menschlichen oder murinen Ursprungs ist oder sogar selbst eine Xenohybridzelle (Mensch/Maus) darstellt.

In analoger Weise, wie es vielfach gelungen ist, B-Lymphozyten mit permanent wachsenden Zellen (ausgewählte Myelomlinien) zu fusionieren, so ist es ebenfalls gelungen, T-Lymphozyten mit geeigneten Fusionspartnern (J.Exp.Med. (1981) 154, 1827-1837) zu verschmelzen. Die von derartigen Hybridomazellen produzierten Stoffe sind, der Natur von T-Zellen entsprechend, Faktoren wie sie von T-Lymphozyten produziert werden können. Derartige Faktoren können z. B. Stoffe wie Interleukine, Interferone, Lymphokine und andere direkt oder indirekt immunregulatorisch wirkende Stoffe sein. Vergleichbar zur B-Lymphozytenfusion können solche Hybride ebenfalls als Produktionsquelle für diese Faktoren benutzt werden, wobei durch geeignetes Selektions- und Testverfahren solche Hybridzellen kloniert und weitergezüchtet werden können, die ausschließlich, oder in besonders großer Menge, die gewünschten Stoffe produzieren.

Ebenso wie für die Fusion von B-Lymphozyten mit Myelomzellen haben sich für T-Lymphozyten bisher nur wenige permanente Zellinien als geeignete Fusionspartner erwiesen. Als geeignete Fusionspartner sind solche permanent wachsende Zellinien anzusehen, die in der Lage sind, mit der entsprechenden Partnerzelle zu fusionieren und nach Fusion als Hybridzelle die gewünschten Stoffe zu sekretieren und sowohl von der Partnerzelle als auch von den aus der Fusion resultierenden Hybridzellen separierbar sind. Prinzipiell kann zur Separation von Hybridzellen von den entsprechenden Fusionspartnern jegliches Unterscheidungskriterium (z. B. unterschiedliche Marker, wie sie auch Oberflächenantigene darstellen) ausgenutzt werden. Als besonders geeignetes Selektionsverfahren hat sich jedoch ein Prinzip herausgestellt, bei dem veränderte Wachstumseigenschaften der Hybridzelle gegenüber den Fusionspartnern ausgenützt werden. Zu diesem Zweck kann man als Zelle, die ein permanentes Wachstum gewährleisten soll und in ihren Wachstumseigenschaften verändert ist, beispielsweise eine Mutante einer permanenten Zellinie verwenden, die einen bestimmten Enzymeffekt hat, indem sie beispielsweise im Vergleich zur Mutterzelle nicht in der Lage ist, Hypoxanthin-guanin-phosphoribosyltransferase zu synthetisieren (HGPRT⁻-Mutante). Solche enzym-defizienten Mutanten permanenter Zellinien sind nicht in der Lage, in geeigneten selektiven Hypoxanthin-Aminopterin-Thymidin-Medien (HAT-Medien) zu überleben. Erst ein Hybridom aus einer derartigen enzymdefizienten Mutante und einer Zelle ohne derartigen Defekt, wie es z. B. eine Blutzelle sein kann, ist in der Lage, in einem derartigen Selektivmedium zu überleben. Verglichen mit anderen bekannten Verfahren stellt ein derartiges Selektionsverfahren zur Isolierung von Hybridzellen von Zellen permanent wachsender Zellinien ein außerordentlich effektives Verfahren dar. Voraussetzung zur Verwendung dieses Verfahrens ist jedoch die Verfügbarkeit einer fusionsfähigen permanent wachsenden Zellinie mit geeigneter Enzymdefizienz.

Die vorliegende Erfindung beschreibt die Herstellung einer fusionsfähigen Zellinie, die als Mutante der Zellinie U-937 (Int. J. Cancer (1976) 17, 565-577) nicht in der Lage ist, Hypoxanthin-guanin-phosphoribosyltransferase zu synthetisieren und daher in einem Hypoxanthin-Aminopterin-Thymidin enthaltenden Medium (HAT-Medium) nicht wachsen kann und abstirbt. Im Gegensatz zu den bisher bekannten HGPRT⁻-Zellinien, die als Fusionspartner für Lymphozyten benutzt werden können, kann die in dieser Erfindung beschriebene HGPRT⁻-Mutante der Zellinie U-937 zu einer Fusion mit Monozyten verwendet werden.

Monozyten sekretieren verschiedene Stoffe, von denen es wünschenswert wäre, sie in größerer Menge herstellen zu können wie Interleukine oder Lymphokine und TNF (tumor necrotizing factor). Zu diesem Zweck wären permanent wachsende Monozyten-Hybride geeignet.

Wahrend die Kenntnisse über B-Lymphozyten verglichen mit denen über andere immunkompetente Zellen sehr groß ist, ist die Erforschung der Eigenschaften von T-Lymphozyten und Monozyten in Nagetieren und in Menschen durch die folgenden Gegebenheiten erschwert:
Obwohl in T-Lymphozyten und Monozyten gewisse funktionelle Aktivitäten ausgelöst werden können, verlangt diese Stimulierung Maßnahmen, die weitere nicht spezifische Effekte auslösen können. Weiterhin ist die Reproduzierbarkeit der Stimulierung gering und es wird nur eine begrenzte Menge sekretierter Produkte erhalten. Auch kann die Zellpopulation, die stimuliert wird, nicht exakt definiert werden, da lediglich eine kleine Zahl von Zellen mit einer begrenzten Wachstumsrate verfügbar ist. Die Anwesenheit selbst einer kleinen Zahl anderer Zelltypen kann nicht vernachlässigt werden angesichts der Tatsache, daß selbst ein kleiner Anteil verunreinigender Zellen erstaunliche biologische Effekte bewirken kann.

Im Gegensatz zu B-Zellhybridzellen ist für Monozytenhybride kein einheitliches Testsystem wie der Immunglobulin-Nachweis möglich. Auf unbekannte Faktoren kann bisher nur in aufwendigen Funktionstestsystemen getestet werden.

Es bestand daher ein Bedürfnis an einer permanenten Zellinie, die es erlaubt, durch Fusion mit Monozyten permanent wachsende Monozytenhybride herzustellen, die diese Schwierigkeiten in der Erforschung von Funktionen von Monozyten überwindet.

Es ist uns überraschenderweise gelungen, durch Behandeln von Zellen der Zellinie U-937 mit Ethyl-methan-sulfonat (EMS) und Behandeln dieser Zellen mit steigenden Konzentrationen von 6-Thioguanin und Abtrennen der jeweils überlebenden Zellen mutierte Zellen zu erhalten, die Hypoxanthin-guanin-posphoribosyltransferase (HGPRT; EC 2.4.2.8.) nicht synthetisieren und in einem Hypoxanthin, Aminopterin und Thymidin enthaltenden Medium (HAT-Medium) nicht wachsen können und absterben.

Gegenstand der Erfindung ist daher eine Mutante der permanenten humanen histiozytischen Zellinie U-937, dadurch gekennzeichnet, daß diese hypoxanthin-guanin-phosphoribosyltransferase-defizient ist und in einem Hypoxanthin, Aminopterin und Thymidin enthaltenden Medium (HAT-Medium) abstirbt.

Zellen dieser Mutante sind geeignet als Fusionspartner von Monozyten. Auf diese Weise hergestellte Hybride sind geeignet, von Monozyten sekretierte Stoffe wie Interleukine oder TNF in Zellkultur zu gewinnen. Die beschriebene Mutante von U-937 kann als Zwischenprodukt zur Gewinnung geeigneter Hybride angesehen werden.

Zur Gewinnung dieser Mutante werden Zellen der Zellinie U-937 in einem Medium wie RPMI 1640 unter Zugabe von Antibiotika und gegebenenfalls fötalem Kälberserum wachsen gelassen. Um ein logarithmisches Wachstum zu gewährleisten, sollte die Konzentration der Zellen $5 \times 10^5$ pro ml nicht übersteigen. Das Medium wurde etwa zweimal pro Woche gewechselt.

- 6 -

Zur Mutation der Zellen wurden sie etwa 12 Stunden lang
in einem Medium inkubiert, das etwa 100 µg/ml Ethyl-
methan-sulfonat enthielt. Danach wurden die Zellen etwa
3 Tage lang in einem normalen Wachstumsmedium vermehrt.

Zur Selektion mutierter Zellen wurde dann einer Suspension der Zellen in RPMI-Medium 0,5 µg 6-Thioguanin/ml
zugesetzt. Wenn mehr als 90 % der Zellen abgestorben
waren, wurde frisches Medium, das 12-O-tetra-decanoyl-
phorbol-13-acetat (TPA) in einer Konzentration von 1.2 x
$10^{-9}$ mol/1 enthielt, über einen Zeitraum von 3 Tagen zugegeben. Das führte zum Haften der lebenden Zellen am
Boden des Kulturgefäßes, während die toten Zellen suspendiert blieben und mit der Flüssigkeit abgeschüttet
werden konnten. Wenn die abgetrennten lebenden Zellen im
folgenden ein exponentielles Wachstum in Anwesenheit von
6-Thioguanin zeigten, wurde die Konzentration des 6-Thio-
guanins verändert und das gerade beschriebene Verfahren
zur Abtrennung der überlebenden Zellen wiederholt. Nach
Behandlung der Zellen mit steigenden Konzentrationen von
6-Thioguanin über einen Zeitraum von etwa 3 Monaten
unter jeweiliger Gewinnung der überlebenden Zellen wurde
eine Zellpopulation erhalten, die in einem Kulturmedium
wuchs, das etwa 65 µg/ml 6-Thioguanin enthielt.

Auf diese Weise gewonnene 6-thioguanin-resistente Zellen
wurden aus einem Kulturmedium, das 65 µg/ml 6-Thioguanin
enthielt, kloniert. Parallel wurde sichergestellt, daß
solche Klone in einem HAT-Medium innerhalb 48 Stunden
abstarben. Aus einer solchen Zellpopulation wurden dann
Einzelzellen kloniert. Solche Klone können dann mit
Monozyten zur Gewinnung von entsprechenden Hybridzellen
fusioniert werden.

Die Zellklone wurden autoradiographisch und szintillationsspektrometrisch auf HGPRT-Aktivität geprüft. Als

Markierungsmittel diente $^3$H-Hypoxanthin. Normale U-937
Zellen bzw. Kulturmedium dienten als positive und negative Kontrollen.

Etwa die Hälfte der U-937 Zellen überlebten die Behandlung mit Ethylmethan-sulfonat und die folgenden 3 Tage
in normalem Kulturmedium. Dies konnte durch den Trypanblau-Ausschlußtest und dadurch gezeigt werden, daß die
Zugabe von TPA die Hälfte der Zellen dazu brachte, sich
am Glasboden anzusetzen. Dies ist eine Eigenschaft lebender Zellen der Zellinie U-937. Diese Methode zeigte
sich ebenfalls als nützlich und zeitsparend für die
Abtrennung überlebender Zellen während der Selektion
mittels des toxischen Analogon 6-Thioguanin. Die Klonierung ergab 3 Klone mit einer Verdopplungszeit von 48
Stunden. Diese Klone starben in HAT-Medium ab.

Während die unbehandelten Ausgangszellen von U-937 in
der Autoradiographie eine kräftige Markierung in der
Kerngegend zeigten, zeigten die selektierten Klone aufgrund ihres HGPRT-Mangels kaum eine Markierung. Dies
konnte durch die direkte Messung des $^3$H-Hypoxanthin-
Aufnahme bestätigt werden. Die 3 mutierten Klone zeigten
maximal 4,1 % des Wertes unbehandelter U-937 Zellen.

Zur Bestimmung des Phenotyps der mutanten Klone wurde
mit entsprechenden Substraten auf die Exprimierung von
saurer Phosphatase, saurer Esterase und einer Esterase
geprüft, die Naphthol AS-D Chloressigsäureester spaltet.
Weiterhin wurde die Isoenzymstruktur der exprimierten
sauren Phosphatase und Esterase mittels isoelektrischer
Fokussierung bestimmt.

Die Reativität der Klone mit verschiedenen monoklonalen
Antikörpern wurde an tiefgefrorenen Zytozentrifugations-
Präparationen mit Hilfe eines indirekten Enyzm-Immuno-Assays mit alkalischer Phosphatase als Enzym bestimmt.
Als T-zellspezifische Antikörper wurden die Antikörper des Standes der Technik OKT 3, 4, 8 und 11 (Ortho
Pharmaceuticals, Raritan, New Jersey, USA) und für die
Bestimmung B-zellassoziierter Antigene der Antikörper
To 15 (Fa. Dako, Santa Barbara, Californien, USA), der
ein allgemeines Reagenz für B-Zellen mit Ausnahme von
Plasmazellen ist, verwendet. Weiterhin wurden verwendet
der monoklonale Antikörper OKM ebenfalls von Ortho,
Anti-human-Monozyt 1 und 2 sowie die monoklonalen Antikörper der Ki-M Serie, nämlich Ki-M 1-8 (Blood (1983)
62, 585; J.Immunol. (1983) 131, 2719; Cell.Immunol.
(1983) 82, 174; Am.J. Pathol. (1984) 117, 441).

Der Phenotyp der U-937-Mutanten-Klone wurde verglichen
mit den ursprünglichen U-937 Zellen sowie mit normalen
menschlichen Blutmonozyten und peritonealen Makrophagen.
Es wurden keine wesentlichen Unterschiede im Phenotyp
der mutanten Klone gefunden, weder zu dem der unbehandelten U-937 Zellen noch dem von anderen bekannten
enzym-defizienten Klone von U-937-Zellen.

Morphologisch zeigen die neuen Zellklone einen gerundeten Zellkern und ein mittelgroßes Zytoplasma. Sie
zeigen keine Napthol AS-D-Chloracetatase- oder Myeloperoxidase-Aktivität. Die Prüfung auf saure Esterase
und saure Phosphatase ergab ein diffuses oder granuläres
Reaktionsmuster von hoher Intensität.

Isoelektrische Fokussierung ergab, daß die von den neuen
Klonen exprimierte saure Esterase aus 5 Isoenzymen und
die exprimierte saure Phosphatase aus 11 Isoenzymen
bestand. Die Muster waren nicht unterscheidbar von denen
normaler humaner Monozyten, während peritoneale Makrophagen zusätzlich zum Grundmuster von Monozyten zwei zusätzliche Banden im Muster der sauren Esterase zeigten.

Während die neuen Klone sowie die ursprünglichen U-937 Zellen lediglich mit den monoklonalen Antikörpern Ki-M 3, Ki-M 7 sowie OKM reagieren, ist bekannt, daß Blutmonozyten und die Mehrzahl von Gewebemakrophagen mit den Antikörpern Ki-M 1, 2, 5, 6, 7 und 8 sowie Mono 1 und Mono 2 reagieren.

Die hier beschriebenen HGPRT-defizienten Klone sind bisher länger als ein halbes Jahr bezüglich ihrer Wachstumseigenschaften, ihres Enzymmangels und ihrer Phenotyp-Charakteristika stabil gewesen.

Die hier beschriebenen neuen Zellklone können als ein in vitro-Äquivalent menschlicher Monozyten/Makrophagen-Zellinien angesehen werden. Die enge Verwandtschaft von humanen Monozyten/Makrophagen und U-937 Zellen ist bekannt.

Zellen der beschriebenen Klone wurden in der klassischen, für B-Zellhybride beschriebene Methode (Nature (1975) 256, 495) in Anwesenheit von PEG mit aus peripherem Blut isolierten Human-Monozyten fusioniert. Unter dem Begriff Monozyt sollen alle Differenzierungsstufen vom Knochenmark bis zum peripheren Blut bzw. Gewebe verstanden werden. Die resultierenden Hybridzellen wiesen die zu erwartenden morphologische Merkmale des Monozyten auf. Die erste Selektion dieser Hybride von den Fusionspartnern beruhte auf ihrer Fähigkeit in HAT-Medium permanent zu wachsen.

Außer durch morphologische Charakteristika wurden diese Hybridklone ebenfalls durch ihre Fähigkeit monozytenspezifische Faktoren zu produzieren als Monozyten-Hybride charakterisiert. So konnte im Zellkulturüberstand eines derartig hergestellten Monozytenhybrids Interleukin-1 Aktivität durch das in der Literatur beschriebene Interleukin-1-Testsysteme (Immunobiol. (1984) 166, 318-333) nachgewiesen werden. Interleukin-1 wird nach der in der Literatur vorherrschenden Meinung unter physiologischen Bedingungen nur von aktivierten Monozyten ausgeschieden.

Analog können Hybride hergestellt werden, die andere Faktoren sekretieren.

<u>Patentansprüche</u>:

1. Mutierte Zellen der permanenten humanen histiozytischen Zellinie U-937, dadurch gekennzeichnet, daß
diese hypoxanthin-guanin-phosphoribosyltransferase-
defizient sind und in einem Hypoxanthin, Aminopterin
und Thymidin enthaltenden Medium (HAT-Medium) absterben.

2. Verwendung der Zellen nach Anspruch 1 als Fusionspartner von Monozyten.

3. Verfahren zur Herstellung mutierter Zellen nach Anspruch 1, dadurch gekennzeichnet, daß Zellen der
Zellinie U-937 mit einem zur Mutierung tierischer
Zellen bekannten chemischen Agenz, vorzugsweise Ethyl-
methan-Sulfonat, und dann mit Konzentrationen von
6-Thioguanin, die stufenweise von 0,5 µg/ml bis mindestens 50 µg/ml steigen, behandelt werden, wobei
jeweils die überlebenden Zellen abgetrennt und in die
folgende Stufe eingesetzt werden und die schließlich
erhaltenen Zellen, in denen praktisch keine Hypoxanthin-guanin-phosphoribosyltransferase exprimiert
wird, vermehrt werden.

4. Zellhybrid erhalten durch Fusion einer Zelle nach Anspruch 1 mit einem Monozyten.